(19) Europäisches Patentamt
European Patent Office
Office européen des brevets

(11) Veröffentlichungsnummer : **0 452 267 A1**

# (12) EUROPÄISCHE PATENTANMELDUNG

(21) Anmeldenummer : **91810244.3**

(51) Int. Cl.[5] : **C07D 295/03,** A01N 43/40

(22) Anmeldetag : **03.04.91**

(30) Priorität : **12.04.90 CH 1264/90**

(43) Veröffentlichungstag der Anmeldung :
**16.10.91 Patentblatt 91/42**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Isenring, Hans Peter, Dr.**
**Himmelrainweg 5**
**CH-4450 Sissach (CH)**
Erfinder : **Pfiffner, Albert, Dr.**
**Grampenweg 10**
**CH-8180 Bülach (CH)**

## (54) Heterocyclische Verbindung.

(57) Es wird das (S)-Enantiomere von Fenpropidin, (S)-1-[3-(p-tert.Butylphenyl)-2-methylpropyl]-piperidin, und dessen Säureadditionssalze und deren Herstellung beschrieben. Das (S)-Enantiomere und seine Säureadditionssalze besitzen fungizide Eigenschaften und eignen sich somit als Wirkstoffe von fungiziden Mitteln zur Verwendung bei der Bekämpfung von Fungi in der Landwirtschaft und im Gartenbau.

EP 0 452 267 A1

Heterocyclische Verbindung

Die vorliegende Erfindung betrifft ein neues optisch aktives Phenylpropylpiperidinderivat, und zwar das (S)-Enantiomere von Fenpropidin, nämlich das (S)-1-[3-(p-tert.Butylphenyl)-2-methylpropyl]-piperidin der Formel

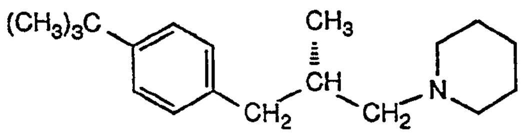

I

sowie die Säureadditionssalze dieses Enantiomeren.

Aus verschiedenen Literaturstellen ist das Racemat, also die RS-Form, von 1-[3-(p-tert.Butylphenyl)-2-methylpropyl]-piperidin (Fenpropidin selber) bekannt geworden. Beispielsweise wird dieses Racemat bereits in der am 25. Mai 1978 veröffentlichten deutschen Offenlegungsschrift (DOS) Nr. 2.752.135 beschrieben, und zwar ohne Nennung der R- und S-Enantiomeren. Die Verwendung des Racemats und von weiteren strukturell verwandten Phenylpropylpiperidinen und -morpholinen als Fungizide in der Landwirtschaft und im Gartenbau wird hier ebenfalls beschrieben. In den Artikeln "Stereoselectivity of Pesticides", Ariens, van Rensen und Welling (Herausgeber), Elsevier 1988, und "The Mode of Action of Morpholines" von E.I. Mercer aus Sterol Biosynthesis Inhibitors, D. Berg und M. Plempel (Herausgeber), VCH-Verlag/Ellis Horwood 1988, wird darauf hingewiesen, dass Fenpropidin ein Chiralitätszentrum an mittleren Kohlenstoffatom der Trimethylenkette hat und dass deswegen grundsätzlich zwei Enantiomere (R- und S-) möglich sind. Das Handelsprodukt Fenpropidin sei ein RS-Enantiomerengemisch. Diese Fachliteratur enthält jedoch keinen Hinweis auf die Herstellbarkeit der beiden Enantiomeren.

Allerdings wurde bereits 1979/80 der Versuch unternommen, das strukturähnliche Handelsfungizid Fenpropimorph [d.h. 1-[3-(p-tert.Butylphenyl)-2-methylpropyl]-cis-3,5-dimethylmorpholin] in sein (S-)-Enantiomeres und sein (R)-Enantiomeres aufzutrennen, in der Hoffnung, dass eines der beiden eine deutlich günstigere Fungizidwirkung als das andere erzielen würde. In der Tat erzielt das (S)-Enantiomere gegenüber dem (R)-Enantiomeren eine leicht höhere Wirkung im Rahmen der üblichen Erwartung. Diese etwas stärkere Wirkung ist indessen, wie auch weiter unten gezeigt werden kann, für praktische Bedürfnisse der Entwicklung eines eigenen auf (S)-Fenpropimorph basierenden Handelspräparats unzureichend. Es ist daher auch im Verlaufe der nachfolgenden Jahre kein Fungizid auf dem Markt erschienen, bei dem allein (S)-Fenpropimorph den bestimmenden Wirkungsanteil dargestellt hätte.

In Analogie dazu konnte ein Fachmann dementsprechend auch vermuten, dass sich das racemische Fenpropidin in präparativ gleicher Weise in das fungizid etwas stärker wirkende (S)-Fenpropidin und das etwas weniger aktive (R)-Fenpropidin würde trennen lassen, ohne jedoch einer solchen Trennung grössere Erwartungen einer deutlich verbesserten Bekämpfung von Pflanzenkrankheiten entgegenzubringen.

Es hat sich indessen überraschenderweise gezeigt, dass sich die bei der racemischen Spaltung des (R,S)-Fenpropimorph publizierten Erfahrungen ganz und gar nicht auf das strukturchemisch analoge (R,S)-Fenpropidin und das biologische Verhalten seiner Enantiomeren übertragen lassen.

Versuche, das gewünschte (S)-1-[3-(p-tert.Butylphenyl)-2-methylpropyl]-piperidin analog der in der Europäischen Patentpublikation EP-A-14 999 bzw. der Deutschen Offenlegungsschrift Nr. 2.907.614 für Fenpropimorph [(S)-1-[3-(p-tert.Butylphenyl)-2-methylpropyl]-cis-3,5-dimethylmorpholin] beschriebenen Racematspaltung mit (+)-Campher-10-sulfonsäure herzustellen, scheiterten. Bei der Kristallisation wurde keine Anreicherung eines enantiomeren Salzes gefunden. Auch weitere, für Racematspaltungen gebräuchliche optische aktive Säuren wurden eingesetzt, um trennbare diastereomere Salze zu erhalten. Im Falle der Verwendung der gleichfalls erwähnten D(-)-Weinsäure an racemischem Fenpropidin erfolgte in den Lösungsmitteln Aceton, Aethylacetat und Aethanol keine Kristallisation, und es bestand damit keine Isolierbarkeit. Bei der Verwendung von Toluol als Lösungsmittel ergab die fraktionierte Kristallisation nur Mischkristalle. Dasselbe Ergebnis trat bei der Verwendung von (+)-Di-O,O-p-toluyl-D-weinsäure als optisch aktive Säure und von Aceton, Aethylacetat, Aethanol oder Toluol als Lösungsmittel ein. Bei der Verwendung von D(-)-Mandelsäure, L-Pyroglutaminsäure, D(-)-Isoascorbinsäure oder Abietinsäure als optisch aktive Säure, die z.T. gleichfalls in der EP-A-14 999 genannt werden, und Aethanol als Lösungsmittel wurde überhaupt keine Kristallisation erzielt. Auch ein Versuch mit (-)-Diaceton-2-keto-L-gulonsäure und Aceton führte zu keiner Kristallisation und demzufolge zu keiner erfolgreichen Racematspaltung von (RS)-1-[3-(p-tert.Butylphenyl)-2-methylpropyl]-piperidin.

Es wurde nun überraschenderweise gefunden, dass (S)-1-[3-(p-tert.Butylphenyl)-2-methylpropyl]-piperidin und dessen Säureadditionssalze erstmalig hergestellt werden können, indem man das bekannte (S)-Fenpropimorph, (S)-1-[3-(p-tert.Butylphenyl)-2-methylpropyl]-cis-3,5-dimethylmorpholin, mit einem Chlorameisensäure-niederalkylester umsetzt und das so erhaltene (S)-1-tert.Butyl-4-(3-chlor-2-methylpropyl)-benzol mit Piperidin umsetzt, und gewünschtenfalls das so hergestellte (S)-1-[3-(p-tert.Butylphenyl)-2-methylpropyl]-piperidin mit einer Säure zum entsprechenden Säureadditionssalz umsetzt. Das so erhaltene Produkt [(S)-Fenpropidin oder ein Säureadditionsalz davon] lässt sich im wesentlichen in reiner Form isolieren, es liegt im wesentlichen frei vom entsprechenden (R)-Enantiomeren vor, und ist zudem als Säureadditionssalz in kristalliner Form zugänglich. Dieser erstmalige Zugang bildet einen weiteren Gegenstand der vorliegenden Erfindung.

Als Säureadditionssalze des erfindungsgemässen (S)-1-[3-(p-tert.Butylphenyl)-2-methylpropyl]-piperidins kommen physiologisch verträgliche Salze in Frage. Hierzu gehören vorzugsweise Salze oder Additionsprodukte dieses Enantiomeren mit anorganischen und organischen Säuren, wie Salzsäure; Bromwasserstoffsäure, Salpetersäure; Phosphorsäure; mono- und bifunktionellen Carbonsäuren und Hydroxycarbonsäuren, z.B Essigsäure, Propionsäure, Maleinsäure, Adipinsäure, Bernsteinsäure, Fumarsäure, Weinsäure, Zitronensäure, Salicylsäure, Sorbinsäure und Milchsäure; und Sulfonsäuren, z.B. Sulfaminsäure und 1,5-Naphthalindisulfonsäure.

Das erfindungsgemässe Verfahren ist zweistufig mit einer fakultativen Endstufe, bei der es sich um die gewünschte Salzbildung handelt. In der ersten Stufe wird (S )-1-[3-(p-tert.Butylphenyl)-2-methylpropyl]-cis-3,5-dimethylmorpholin, also (S)-Fenpropimorph der Formel

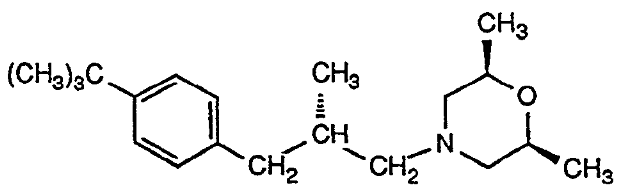

II

mit einem Chlorameisenäsure-niederalkylester der Formel

ClCOOR III

worin R eine Niederalkylgruppe, insbesondere $C_{1-4}$-Alkyl, bedeutet, umgesetzt, und zwar zum (S)-1-tert.Butyl-4-[3-chlor-2-methylpropyl]benzol der Formel

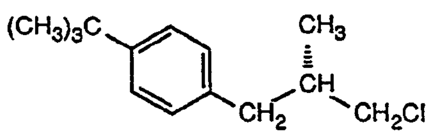

IV

Falls R der Formel III $C_3$ oder $C_4$-Alkyl bedeutet, kann dies geradkettig oder verzweigt sein. R ist jedoch vorzugsweise Aethyl. In den obigen Formeln II und IV (und auch I) stellt die Teilstruktur

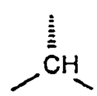

jeweils das (S)-Chiralitätszentrum dar.

Diese Umsetzung erfolgt zweckmässigerweise in einem inerten lösungsmittel, wie beispielsweise n-Hexan, einem halogenierten Kohlenwasserstoff, z.B. Chloroform, oder einem Aromat, z.B. Benzol, Toluol oder Nitrobenzol, bei Temperaturen zwischen 5°C und der Rückflusstemperatur des Reaktionsgemisches. Das Produkt der Formel IV kann nach an sich bekannten Methoden, z.B. Säulenchromatographie oder Destillation, isoliert und gereinigt werden.

Bei der Spaltung von tertiären Aminen nach einer an sich bekannten Methode [T. Kometani, S. Shiotani und K. Mitsuhashi, Chem. Pharm. Bull. 24(2), 342 (1976)] besteht das Ziel darin, Carbamate herzustellen oder die Ringöffnung von Tetrahydroisochinolinen zu realisieren. Die für den vorliegenden Zweck interessante Chlorverbindung der Formel IV wird in dieser Literaturstelle nur als Nebenprodukt beschrieben. Ferner wird behauptet, dass die Reaktivität der Amine gegenüber Aethylchlorformat mit zunehmender Grösse der Gruppen um das Stickstoffatom abnimmt. Die Autoren geben an, dass die Methode ziemlich selektiv für die Spaltung von Benzyl-N, Allyl-N und Methyl-N-Bindungen ist. In einer weiteren Publikation [H. Al Saadi, G. Ricart und D. Couturier, C. R. Acad. Sc. Paris, t. 286, Série C 469 (1978)] wird erwähnt, dass die Methode hauptsächlich zur Herstellung sekundärer Amine via Carbamate geeignet ist, und daneben auch zur Herstellung von Allylchloriden. Es wird angegeben, dass in cyclischen Aminen der 5-gliedrige Ring sehr leicht, der 6-gliedrige Piperidinring jedoch schwer gespalten wird. In einer dritten Publikation [R. Mornet und L. Gouin, Synthesis, 786 (1977)] wird ebenfalls die Herstellung von Allylchloriden mit dieser Methode beschrieben. Ueberraschenderweise gelang es, gerade diese Methode zur Spaltung von tertiären Aminen für die erste Stufe des erfindungsgemässen Verfahrens anzuwenden.

Das so erhaltene (S)-1-tert.Butyl-4-[3-chlor-2-methylpropyl]-benzol IV wird in der zweiten Reaktionsstufe mit Piperidin zum gewünschten (S)-1-[3-(p-tert.Butylphenyl)-2-methylpropyl]-piperidin I umgesetzt. Dies erfolgt zweckmässigerweise ohne Gegenwart von zusätzlichem Lösungsmittel (das Piperidin dient sowohl als Reaktionspartner als auch als Lösungsmittel) bei der Rückflusstemperatur des Reaktionsgemisches. Wegen der Doppelrolle des Piperidins wird dies zweckmässigerweise im Ueberschuss eingesetzt. Man kann den Reaktionsverlauf beispielsweise durch Gaschromatographie verfolgen und nötigenfalls zusätzliche Mengen Pyridin zum Reaktionsgemisch geben. Die Isolierung und Reinigung des Produktes I kann nach an sich bekannten Methoden, z.B. Säulenchromatographie oder Destillation, erfolgen.

Zur Herstellung eines Säureadditionssalzes des (S)-1-[3-(p-tert.Butylphenyl)-2-methylpropyl]-piperidins wird dies mit der gewünschten Säure auf übliche Weise umgesetzt. Das Salz kann nach an sich bekannten Methoden isoliert und gereinigt werden. Als Alternative kann die Herstellung eines Säureadditionssalzes gegebenenfalls "in situ" bei der Formulierung erfolgen.

Das erstmalig nach der vorstehend beschriebenen Methode gewonnene erfindungsgemässe (S)-Fenpropidin oder (S)-1-[p-tert.Butylphenyl)-2-methylpropyl]-piperidin, das ein Gegenstand dieser Erfindung ist, und dessen Säureadditionssalze (im folgenden als erfindungsgemässe Verbindungen oder Wirkstoffe bezeichnet) besitzen unerwarteterweise eine fungizide Wirkung, die ein Mehrfaches von derjenigen des racemischen (R,S)-Fenpropidins darstellt und die die Wirkung des enantiomeren (R)-Fenpropidins im Durchschnitt um einen Faktor von ca. 7-23 übersteigt Den biologischen Werten, wie sie für das strukturähnliche (S)-Fenpropimorph in der EP-A-14 999 angegeben werden, und die sich nur etwa im Bereich von 1.5:1 (S-Racemat) bis 2:1 (S:R) bewegen, konnten keine Hinweise auf die Möglichkeit einer derart sprunghaften Wirkungssteigerung entnommen werden. Viel eher musste vermutet werden, dass auch die biologischen Wirkungsveränderungen beim Fenpropidin denen des Fenpropimorphs ähneln würden. Es bestand daher für den Fachmann keine Veranlassung, nach Wegen zur Gewinnung von Enantiomeren des Fenpropidins zu suchen.

Aufgrund dieses nicht vorhersehbaren Ergebnisses aber liegt mit der vorliegenden Erfindung eine ganz wesentliche Bereicherung der Technik vor.

Das erfindungsgeinässe (S)-Fenpropidin und dessen Säureadditionssalze können als höherqualifizierte Pflanzenfungizide dementsprechend zur Bekämpfung von Pilzen in der Landwirtschaft und im Gartenbau Verwendung finden. Sie eignen sich insbesondere zur Hemmung des Wachstums oder zur Vernichtung von phytopathogenen Pilzen auf Pflanzenteilen, z. B. Blättern, Stengeln, Wurzeln, Knollen, Früchten oder Blüten, und auf Saatgut sowie von im Erdboden auftretenden Schadpilzen.

Die erfindungsgemässen Verbindungen eignen sich besonders zur Bekämpfung von Erysiphe gramminis, Uncinula necator, Sphaerotheca pannosa, Erysiphe betae und Rostkrankheiten, wie beispielsweise solche der Gattungen Puccinia, Uromyces und Hemileia (insbesondere Puccinia recondita, Puccinia striformis, Puccinia graminis, Puccinia coronata, Uromyces fabae, Uromyces appendiculatus, Hemileia vastatrix). Ferner wirken die erfindungsgemässen Verbindungen gegen Schadpilze der Gattungen Helminthosporium (z.B. Helminthosporium oryzae, Helminthosprium teres, Helminthosporium sativum, Helminthosporium tritici repentis), Alternaria (z.B. Alternaria brassicola, Alternaria brassicae), Septoria (z.B. Septoria avenae), Cercospora (z.B. Cercospora beticola) und Ceratocystis (z.B. Ceratocystis ulmi).

Im Freiland werden bevorzugt Dosierungen von 75 bis 1000 g Wirkstoff pro Hektar und Behandlung zur Anwendung gebracht. Zur Bekämpfung von Pilzen im Samenbeizverfahren werden mit Vorteil Dosierungen von 0,05 g bis 1,0 g Wirkstoff pro kg Samen verwendet.

Die erfindungsgemässen Verbindungen zeichnen sich durch systemische, kurative und vorbeugende Wirkung aus. Durch Sekundärverteilung der Wirkstoffe in der Gasphase können teilweise auch nicht direkt behandelte Pflanzenteile geschützt werden.

Die erfindungsgemässen Verbindungen können zu verschiedenartigen Mitteln, z.B. lösungen, Suspensionen, Emulsionen, emulgierbaren Konzentraten und pulverförmigen Präparaten, formuliert werden, und solche fungiziden Mittel bilden einen weiteren Gegenstand der vorliegenden Erfindung. Die erfindungsgemässen fungiziden Mittel sind dadurch gekennzeichnet, dass sie eine wirksame Menge von (S)-1-[3-(p-tert.Butylphenyl)-2-methylpropyl]-piperidin oder eines Säureadditionssalzes davon sowie Formulierungshilfsstoffe enthalten. Bereits ein Wirkstoff-Anteil von mindestens 60 Gew.-% (S)-Enantiomerem im eingesetzten Fenpropidin ergibt eine spürbare Wirkungsverbesserung gegenüber einem fungiziden Mittel, das nur auf racemischem Fenpropidin basiert. Die vorliegende Erfindung bezieht sich ausdrücklich auf fungizide Zusammensetzungen mit durch (S)-Fenpropidin angereichertem bzw. alleinigem Wirkstoff-Anteil. Bevorzugt in der Praxis sind Gewichtsmengenanteile von mindestens 75 %, vorzugsweise mindestens 85 % (S)-Enantiomerem. Die besten Ergebnisse

liefert ein Wirkstoff, der im wesentlichen (92 % oder mehr) nur aus (S)-Fenpropidin besteht und nur geringe oder keine Anteile von (R)-Fenpropidin aufweist. Die Mittel enthalten zweckmässigerweise zumindest einen der folgenden Formulierungshilfsstoffe:

Feste Trägerstoffe; Lösungs- bzw. Dispersionsmittel; Tenside (Netzmittel und Emulgatoren); Dispergiermittel (ohne Tensidwirkung); und andersartige Zusatzstoffe, wie beispielsweise Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoffe und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, höher alkylierte Benzole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester, Ketone, wie Aceton, Methyläthylketon, Methylisobutylketon, Isophoron und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase wie Halogenkohlenwasserstoffe. Im Falle der Benutzung von Wasser als Lösungsmittel können z.B. auch organische Lösungsmittel als Hilfslösungsmittel verwendet werden.

Die Tenside (Netzmittel und Emulgatoren) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockcopolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylaminoniumchloride, Alkyltrimethylammoniumchloride und äthoxylierte quaternäre Ammoniumchloride.

Als Dispergiermittel (ohne Tensidwirkung) kommen im wesentlichen in Frage: Natrium- und Ammoniumsalze von Ligninsulfonsäure, Natriumsalze von MaleinsäureanhydridDiisobutylen-Copolymeren, Natrium- und Ammoniumsalze von sulfonierten Polykondensationsprodukten aus Naphthalin und Formaldehyd, Natriumsalze von polymeren Carbonsäuren und Sulfitablaugen.

Als Dispergiermittel, die sich insbesondere als Verdickungs- und Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylnitrilsäureester und Zimtsäureester, und Deaktivatoren, z.B. Salze der Aethylendiamintetraessigsäure und Polyglykole.

Die erfindungsgemässen fungiziden Mittel können neben dem erfindunsgemässen Wirkstoff auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 95 Gewichtsprozent an erfindungsgemässer Verbindung als Wirkstoff. Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigen Bereich des obigen Konzentrationsintervalls. Emulgierbare Konzentrate enthalten im allgemeinen 5 bis 95 Gewichtsprozent, vorzugsweise 25 bis 85 Gewichtsprozent, der erfindungsgemässen

Verbindung. Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man eine erfindungsgemässe Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netzmittel oder Emulgatoren oder von Dispergiermitteln, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffes imprägnieren und dann das Lösungs-bzw. Dispersionsmittel durch Abdunsten, Erhitzen oder durch Absaugen unter vermindertem Druck entfernen. Durch Zusatz von Tensiden bzw. Dispergiermitteln kann man solche pulverförmige Mittel mit Wasser leicht benetzbar machen, so dass sie in wässrige Suspensionen, die sich z.B. als Spritzmittel eignen, überführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelösten Emulgator enthält, so dass die Lösung bei Zugabe zu Wasser selbstemulgierend wirkt. Andernfalls kann der Wirkstoff mit einem Emulgator vermischt und das Gemisch dann mit Wasser auf die gewünschte Konzentration verdünnt werden. Zudem kann der Wirkstoff in einem Lösungsmittel gelöst und danach mit einem Emulgator gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Die nachstehenden Beispiele illustrieren die Erfindung.

I. Herstellung von (S)-1-(3-(p-tert.Butylphenyl)-2-methylpropyl]piperidin:

Beispiel 1:

(a) (S)-1-tert.Butyl-4-(3-chlor-2-methylpropyl]-benzol

41,4 g (0,136 Mol) (S)-Fenpropimorph werden in 560 ml Toluol gelöst, und die Lösung wird mit 1,4 g (0,01 Mol) Kaliumcarbonat versetzt. Bei einer Innentemperatur von 94°C werden unter intensivem Rühren insgesamt 177 g ( 1,63 Mol) Chlorameisensäure-äthylester in drei Portionen während jeweils 1 Stunde zugetropft (1/3 zu Beginn, 1/3 nach 1 Stunde und 1/3 nach 4 Stunden). Der Reaktionsverlauf wird durch Kapillar-Gaschromatographie verfolgt. Nach einer totalen Reaktionszeit von 6 Stunden dampft man das Gemisch ein und giesst den Rückstand in ein Gemisch von Eiswasser und Essigsäure-äthylester. Man schüttelt und trennt die Phasen. Die Wasserphase wird nochmals mit 500 ml Essigsäure-äthylester extrahiert. Die vereinigten organischen Phasen werden über wasserfreiem Natriumsulfat getrocknet und eingeengt. Man chromatographiert den Rückstand an 700 g Kieselgel 60 (MERCK, Korngrösse 0,040-0,063 mm) mit Essigsäure-äthylester/n-Hexan (1:9). Die reinen Fraktionen werden vereinigt und unter Hochvakuum (Sdp. ca. 80°C) destilliert. Man erhält ein farbloses Oel.

| | |
|---|---|
| Opt. Drehung (c=1 %, $C_2H_5OH$ 96 %): | 365 nm + 70,5° |
| | 436 nm + 44,4° |
| | 546 nm + 25,8° |
| | 578 nm + 23,0° |
| | 589 nm + 22,0° |

(b) (S)-1-(3-(p-tert.Butylphenyl)-2-methylpropyl]-piperidin

27 g (0,12 Mol) (S)-1-tert.Butyl-4-[3-chlor-2-methylpropyl]-benzol werden in 70 ml (0,708 Mol) Piperidin 16 Stunden unter intensivem Rühren auf Rückflusstemperatur erhitzt. Anschliessend giesst man weitere 30 ml (0,303 Mol) Piperidin zu. Der Reaktionsverlauf wird mit Gaschromatographie verfolgt. Man rührt das Reaktionsgemisch eine weitere Stunde bei Rückflusstemperatur und lässt es abkühlen. Man giesst auf 350 ml 2N Natronlauge und extrahiert mit drei Portionen, insgesamt 600 ml, n-Hexan. Die organischen Phasen werden vereinigt, über wasserfreiem Natriumsulfat getrocknet und eingeengt. Man chromatographiert den Rückstand an 700 g Kieselgel 60 (MERCK, Korn-grösse 0,040-0,063 mm) mit Essigsäure-äthylester/n-Hexan (1:1). Die reinen Fraktionen werden vereinigt und unter Hochvakuum (Sdp. ca. 140°C) destilliert. Man erhält ein farbloses Oel.

| | |
|---|---|
| Opt. Drehung (c=1 %, $C_2H_5OH$ 96 %): | 365 nm + 11,1° |
| | 436 nm + 9,0° |
| | 546 nm + 6,0° |
| | 578 nm + 5,5° |
| | 589 nm + 5,4° |

Unter Zugabe des "chiralen Lösungsmittels" (R)-(-)-2,2,2-Trifluor-1-(9-anthryl)-äthanol (TAE) lässt sich im NMR-Spektrum ($CDCl_3$) die optische Reinheit bestimmen. Die Verschiebungen der Signale der Methylgruppe am Chiralitätszentrum ist von der relativen Menge von TAE in der Lösung abhängig: Es gibt keinen Hinweis auf Racemisierung.

II. Formulierungsbeispiele

Beispiel 2: Ein Spritzpulver enthält beispielsweise folgende Bestandteile:

| | Gewichtsprozent |
|---|---|
| Erfindungsgemässe Verbindung I (Wirkstoff) | 25 |
| Kieselsäure hydratisiert (Hilfsträger) | 20 |
| Natrium-laurylsulfat (Netzmittel) | 2 |
| Natrium-lignosulfonat (Dispergiermittel) | 4 |
| Kaolin (Tonerde, Trägerstoff) | 49 |

Insbesondere die erfindungsgemässe Verbindung als flüssige freie Base kann mit dieser Rezeptur als Spritzpulver zubereitet werden.

Zur Herstellung wird vorerst der flüssige Wirkstoff in einem Pulvermischer auf die vorgelegte Kieselsäure aufgedüst. Anschliessend werden die weiteren Komponenten zugemischt, und das Ganze wird unter Verwendung einer Stiftenmühle oder vergleichbaren Mahleinrichtung fein gemahlen.

Beispiel 3:

Erfindungsgemässe Säureadditionssalze mit hohem Schmelzpunkt (etwas ab 100°C) können vorzugsweise auch als höher konzentrierte Spritzpulver formuliert werden, beispielsweise mit folgender Rezeptur:

| | | Gewichtsprozent |
|---|---|---|
| Erfindungsgemässe Verbindung I als Säureadditions-salz (Wirkstoff) | | 75 |
| Kieselsäure hydratisiert (Mahlhilfsmittel) | | 1 |
| Alkylnaphthalinsulfonat und sulfatiertes Alkylcarboxylat als Natriumsalze (Netzmittel) | (1) | 2 |
| Naphthalinformaldehyd-Kondensat, sulfoniert, als Natriumsalz (Disergiermittel) | (2) | 10 |
| Polyvinylpyrrolidon (Bindemittel) | (3) | 1 |
| Kaolin (Tonerde, Trägerstoff) | | 11 |

(1) z.B. Morwet® EFW (De Soto)
(2) z.B. Morwet® D-425 (De Soto)
(3) z.B. PVP K-30 (GAF)

Zur Herstellung werden die Komponenten miteinander gemischt, und das Ganze wird in einer Stiftenmühle oder noch besser in einer Strahlmühle fein gemahlen.

Die resultierenden Spritzpulver ergeben beim Einrühren in Wasser eine feine Suspension in gewünschter Konzentration, die sich als gebrauchsfertige Spritzbrühe eignet.

Beispiel 4:

Ein Spritzpulver, das auf demjenigen des Beispiels 3 basiert, kann auch in ein dispergierbares Granulat übergeführt werden. Dazu wird das gemahlene Pulver in einer geeigneten Granuliervorrichtung (z.B. Granulierteller, Mischtrommel, Intensivmischer oder Wirbelschichtgranulator) mit einer wässrigen Lösung des Bindemittels besprüht, bis sich Agglomerate gebildet haben. Danach wird das zugefügte Wasser in einem Trocknungsprozess wieder entfernt, und die Granulate der gewünschten Partikelgrösse werden ausgesiebt. Das resultierende Granulat hat gegenüber dem Spritzpulver verschiedene Vorteile (keine Staubbildung bei der Applikation, leichtere Dosierbarkeit dank besseren Fliesseigenschaften). Die Applikation erfolgt nach Einrühren des Präparates in Wasser und nach vollständigem Zerfall der Granulate in die Primärpartikel genau gleich wie beim Spritzpulver.

Beispiel 5:

Die kristallinen, gut wasserlöslichen Säureadditionssalze der erfindungsgemässen Verbindung können unter Verwendung löslicher Hilfsstoffe auch als wasserlösliche Pulver und wasserlösliche Granulate formuliert werden, beispielsweise:

| | Gewichtsprozent |
|---|---|
| Erfindungsgemässe Verbindung I als Hydrochlorid (Wirkstoff) | 57 |
| Kieselsäure hydratisiert (Mahlhilfsmittel) | 2 |
| Nonylphenol-polyäthoxyat (Netzmittel) | 10 |
| Harnstoff gemahlen (Trägerstoff) | 31 |

Zur Herstellung werden zunächst der Wirkstoff und der Trägerstoff miteinander gemischt, danach wird das erwärmte Netzmittel auf die Mischung aufgesprüht und die Kieselsäure als Mahlhilfsmittel zugegeben. Diese Mischung wird mit einer üblichen Mahleinrichtung gemahlen.

Das resultierende Pulver ergibt beim Einrühren in Wasser eine Lösung, die sich als gebrauchsfertige Spritzbrühe eignet.

Soll das Pulver zu einem wasserlöslichen Granulat weiterverarbeitet werden, wird das Pulver zunächst mit Wasser leicht befeuchtet und danach in einer geeigneten Granuliervorrichtung (z.B. "Alexander"-Extrusions-Granulator) granuliert und anschliessend getrocknet. Die Applikation erfolgt nach Einrühren in Wasser und nach vollständiger Auflösung genau gleich wie beim Spritzpulver.

Verbindung 6:

Die erfindungsgemässe Verbindung in Form der freien Base ist in den gängigen organischen Lösungsmitteln gut löslich. Durch Zusatz von Emulgatoren lassen sich so emulgierbare Konzentrate herstellen:

| | |
|---|---|
| Erfindungsgemässe Verbindung I (Wirkstoff) | 750 g/l |
| Isotridecanolpolyäthoxylat (nichtionischer Emulgator) | 75 g/l |
| Calcium-dodecylbenzolsulfonat (anionischer Emulgator) | 25 g/l |
| Gemisch von Alkylbenzolen (Lösungsmittel) (4) | 1000 ml |

(4) z.B. SOLVESSO®100 (EXXON) oder Shellsol®A (SHELL)

Der Wirkstoff und die Emulgatoren werden unter Rühren im Gemisch von Alkylbenzolen gelöst. Die resultierende Lösung lässt sich in Wasser emulgieren und ergibt so eine gebrauchsfertige Spritzbrühe in der gewünschten Konzentration.

Beispiel 7:

Ein wasserverdünnbares Konzentrat eines "in situ" hergestellten Säureadditionssalzes erhält man beispielsweise mit:

| | | |
|---|---|---|
| Erfindungsgemässe Verbindung I als freie Base (Wirkstoff) | | 250 g/l |
| Essigsäure (Neutralisierungsmittel) | | 44 g/l |
| Milchsäure (Neutralisierungsmittel) | | 38 g/l |
| Isopropanol (Cosolvens) | | 300 g/l |
| Wasser (Lösungsmittel) | ad | 1000 ml |

Dieses Konzentrat kann zur Applikation mit Wasser im üblichen Rahmen verdünnt werden und ergibt so eine klare bis leicht opale gebrauchsfertige Spritzbrühe.

Beispiel 8: Wachstumshemmung phytopathogener Pilze

Die fungizide Aktivität der Testsubstanzen wird mit Hilfe des Radialwachstumtests bestimmt. Dabei dienen die aus der EP-A-14 999 bekannt gewordenen strukturähnlichen Enantiomeren des Fenpropimorph als Vergleichssubstanzen.

Die verwendeten Pilze sind Pathogene der folgenden Kulturpflanzen:

| | |
|---|---|
| Helminthosporium oryzae | : Reis |
| H. teres | : Gerste |
| H. tritici-repentis | : Weizen |
| H. sativum | : Gerste |
| Septoria avenae | : Hafer |
| Alternaria mali | : Aepfel |
| Alternaria kikuchiana | : Birnen (Jap. Birne) |

A) Methode I:

Es werden acetonische Lösungen abgestufter Konzentration der Testsubstanzen hergestellt, von denen je 20 µl mit 13 ml eines 50°C warmen flüssigen Agarmediums vermengt und in eine Petrischale gegossen werden. Nach dem Erkalten wird der Agar in der Schalenmitte mit Pilz-Inoculum von Helminthosporium oder Alternaria beimpft. Nach der Inoculation werden die Platten inkubiert. Sobald in der unbehandelten Kontrollschale der jeweiligen Testreihe die radiale Ausbreitung des Pilzerregers annähernd den Schalenrand erreicht hat (ca. 5 bis 30 Tage bei H.-Erregern; 9 bis 21 Tage bei Alternaria-Spezies) wird der Durchmesser aller übrigen Pilzkulturen der gleichen Testreihe bestimmt. Die $IC_{50}$-Werte werden daraus graphisch im Verhältnis zur Wirkstoffkonzentration (in µMolen) ermittelt. Für Helminthosporium oder Alternaria wird V-8 Agar verwendet (200 ml V-8 Nährmedium, 3 g $CaCO_3$, 20 g Agar-agar und 200 ml destilliertes Wasser), der einleitend 30 min. im Autoklaven bei 121°C sterilisiert worden war.

Tabelle 1

Wirksamkeit der Enantiomeren von Fenpropidin (FPD) im Vergleich zur Wirksamkeit der Fenpropimorph (FPM)-Enantiomeren

| | $IC_{50}$-Werte in µMol | | | | | |
|---|---|---|---|---|---|---|
| | Fenpropimorph | | | Fenpropidin | | |
| Schadpilz | Racemat | S-FPM | R-FPM | Racemat | S-FPD | R-FPD |
| H. oryzae | 2.1 | 2.7 | 1.4 | 2.4 | 0.35 | 6.1 |
| H. teres | 6.4 | 3.6 | 6.6 | 0.53 | 0.23 | 5.2 |
| H. trit.-rep. | 10.5 | 7.4 | 11.5 | 0.47 | 0.25 | 3.8 |
| H. sativum | 4.6 | 7.2 | 3.6 | 5 | 0.74 | 11.5 |
| A. mali | 3.3 | 2 | 2.75 | 3.5 | 1.7 | 11.5 |
| A. kikuchiana | 6.7 | 7.8 | 11.5 | 9 | 2.85 | 30 |

Aus dem Verhältnis der Messwerte der Tabelle 1 erkennt man den unerwartet hohen Wirkungsunterschied des (S)-Fenpropidins zum (R)-Fenpropidin, der keine Parallele beim Fenpropimorph findet.

Tabelle 2

Verhältnis der $IC_{50}$-Werte der Enantiomeren aus Tabelle 1

| Schadpilz | R-FPM / S-FPM | R-FPD / S-FPD |
|---|---|---|
| H. oryzae | 0.5 | 17.4 |
| H. teres | 1.8 | 22.6 |
| H. trit.-rep. | 1.6 | 15.2 |
| H. sativum | 0.5 | 15.5 |
| A. mali | 1.4 | 6.8 |
| A. kikuchiana | 1.5 | 10.5 |

B) Methode II:

Septoria avenae wird auf Weizenmehl-Agar vorgezüchtet und zu einer Sporensuspension ( $10^6$/ml) verarbeitet. Die acetonischen Wirkstofflösungen werden gemäss Methode I mit warmem Weizenmehl-Agar (20 g Mehl, 1 g Trockenhefe, 20 Agar, 1 Liter Wasser, sterilisiert während 30 min. bei 121°C) vermischt und in Petrischalen abgefüllt. Je 50 µl Sporensuspension werden ins Zentrum des erkalteten Agar gegeben und mit einem Glasstab gleichmässig über die Oberfläche verteilt. Nach ca. 12 Tagen Inkubationszeit werden die punktförmigen Sporenkulturen (plaques) in den Schalen ausgezählt. Im Verhältnis zur Mol-Konzentration des Wirkstoffs werden dann die $IC_{50}$-Werte entsprechend graphisch ermittelt.

Tabelle 3

Wirksamkeit an Septoria avenae der Enantiomeren von Fenpropidin und Fenpropimorph

| $IC_{50}$-Werte in µMol | | | | | |
|---|---|---|---|---|---|
| Fenpropimorph | | | Fenpropidin | | |
| Racemat | S-FPM | R-FPM | Racemat | S-FPD | R-FPD |
| 26 | 26 | 84 | 4.3 | 1.55 | 35 |

Während der Quotient der vorbekannten Isomeren des Fenpropimorph R-FPM/Sp-FPM = 3,2 beträgt, erreicht der entsprechende Quotient bei Fenpropidin R-FPD/S-FPD = 22,6.

**Patentansprüche**

1. (S)-1-[3-(p-tert.Butylphenyl)-2-methylpropyl]-piperidin und dessen Säureadditions salze.

2. (S)-1-[3-(p-tert.Butylphenyl)-2-methylpropyl]-piperidin.

3. Fungizides Mittel das neben einem geeigneten Trägermaterial als Wirkstoff Fenpropidin (= 1-[3-(p-tert.Butylphenyl)-2-methylpropyl]-piperidin) oder dessen Säureadditionssalz enthält, dadurch gekennzeichnet, dass der Anteil des (S)-Enantiomeren mindestens 60 Gewichtsprozent beträgt.

4. Mittel gemäss Anspruch 3, dadurch gekennzeichnet, dass der Anteil des (S)-Enantiomeren mindestens 75 Gewichtsprozent beträgt.

5. Mittel gemäss Anspruch 4, dadurch gekennzeichnet, dass der Anteil des (S)-Enantiomeren mindestens 85 Gewichtsprozent beträgt.

6. Mittel gemäss Anspruch 5, dadurch gekennzeichnet, dass der Wirkstoff Fenpropidin im wesentlichen aus dem reinen (S)-Enantiomeren besteht.

7. Verfahren zur Herstellung von (S)-1-[3-(p-tert.Butylphenyl)-2-methylpropyl]-piperidin und von dessen Säureadditionssalzen, dadurch gekennzeichnet, dass man (S)-1-[3-(p-tert.Butylphenyl)-2-methylpropyl]-Cis-3,5-dimethyl-morpholin mit einem Chlorameisensäure-niederalkylester umsetzt und das so erhaltene (S)-1-tert.Butyl-4-(3-chlor-2-methylpropyl)-benzol mit Piperidin umsetzt, und gewünschtenfalls das so hergestellte (S)-1-[3-(p-tert.Butylphenyl)-2-methylpropyl]-piperidin mit einer Säure zum entsprechenden Säureadditionssalz umsetzt.

8. Verfahren nach Anspruch 7, dadurch gekennzeichnet, dass der Chlorameisensäureniederalkylester Chlorameisensäure-äthylester ist.

9. Verfahren zur Bekämpfung von Pilzen und zur Verhütung von Pilzbefall in der Landwirtschaft und im Gartenbau durch Verwendung von Fenpropidin (= 1-[3-(p-tert.Butylphenyl)-2-methylpropyl]-piperidin) oder dessen Säureadditionssalz, dadurch gekennzeichnet, dass der Wirkstoff mindestens 60 Gewichtsprozent des (S)-Enantiomeren enthält.

10. Verfahren gemäss Anspruch 9, dadurch gekennzeichnet, dass der Wirkstoff mindestens 75 Gewichtsprozent des (S)-Enantiomeren enthält.

11. Verfahren gemäss Anspruch 10, dadurch gekennzeichnet, dass der Wirkstoff mindestens 85 Gewichtsprozent des (S)-Enantiomeren enthält.

12. Verfahren gemäss Anspruch 11, dadurch gekennzeichnet, dass der Wirkstoff Fenpropidin im wesentlichen aus dem reinen (S)-Enantiomeren besteht.

Europäisches Patentamt

# EUROPÄISCHER RECHERCHENBERICHT

Nummer der Anmeldung

EP 91 81 0244

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | DE-A-2 752 135 (F. HOFFMANN-LA ROCHE & CO. AG)<br>* Patentansprüche; Seite 41, Zeilen 1-3 * | 1-6,9-12 | C 07 D 295/03<br>A 01 N 43/40 |
| A,D | EP-A-0 014 999 (BASF AG)<br>* Patentansprüche; Seiten 1-4 * | 7,8 | |
| | | | RECHERCHIERTE SACHGEBIETE (Int. Cl.5) |
| | | | C 07 D 295/00 |

Der vorliegende Recherchenbericht wurde für alle Patentansprüche erstellt

| Recherchenort | Abschlußdatum der Recherche | Prüfer |
|---|---|---|
| DEN HAAG | 17-07-1991 | SANCHEZ Y GARCIA J.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)